**Europäisches Patentamt**

**European Patent Office**

**Office européen des brevets**

(19)

(11) Numéro de publication : **0 498 690 A1**

# (12) DEMANDE DE BREVET EUROPEEN

(21) Numéro de dépôt : **92400192.8**

(22) Date de dépôt : **24.01.92**

(51) Int. Cl.$^5$ : **A61F 2/08**

(30) Priorité : **05.02.91 FR 9101280**

(43) Date de publication de la demande :
**12.08.92 Bulletin 92/33**

(84) Etats contractants désignés :
**DE ES GB IT**

(71) Demandeur : **Surer, Patrick**
**Rue du Prieuré-de-Béré**
**F-44110 Chateaubriant (FR)**

(72) Inventeur : **Surer, Patrick**
**Rue du Prieuré-de-Béré**
**F-44110 Chateaubriant (FR)**

(74) Mandataire : **Moncheny, Michel et al**
**c/o Cabinet Lavoix 2 Place d'Estienne d'Orves**
**F-75441 Paris Cedex 09 (FR)**

(54) **Implant pour chirurgie ligamentaire.**

(57) Cet implant (10) comprend une gaine (12) en un matériau synthétique de préférence résorbable, se terminant à ses deux extrémités par des parties effilées semi-rigides (14), et un greffon naturel (18) disposé à l'intérieur de cette gaine (12). De préférence cette dernière (12) est fendue (16) pour permettre la mise en place du greffon (18) au moment de l'intervention.
Application aux plasties ligamentaires.

FIG.1

EP 0 498 690 A1

La présente invention concerne un implant destiné à être utilisé en chirurgie ligamentaire.

On connaît essentiellement deux types de solutions actuellement utilisées pour reconstruire un ligament. La première consiste à prélever sur le malade, voire dans une banque d'organes, un greffon tendineux ou aponévrotique, puis à le lacer en utilisant des fils que l'on fait dépasser aux deux extrémités du greffon de façon à pouvoir l'introduire dans des tunnels ménagés dans les os du patient. Les fils dépassant aux extrémités facilitent l'introduction dans ces tunnels et permettent d'exercer la traction nécessaire au passage du greffon, puis sa fixation à l'os. Après sa mise en place, le greffon est envahi par le tissu de bourgeonnement, détruit et reconstruit simultanément par l'organisme, de sorte qu'après plusieurs mois il se comporte comme un ligament vivant.

L'inconvénient principal de ce premier type de solution résulte des difficultés opératoires : il est difficile de réaliser un laçage qui ne glisse pas et d'introduire dans un tunnel étroit et rugueux un tissu qui a une tendance spontanée à s'effilocher. De plus la qualité de la fixation est précaire pendant plusieurs semaines, avant le début de la réhabitation dans les tunnels osseux.

Le deuxième type de solution consiste à utiliser des ligaments artificiels qui se présentent sous la forme d'un cordage se terminant à ses deux extrémités par une partie effilée semi-rigide destinée à faciliter l'introduction dans les tunnels osseux et la traction lors du passage dans ces tunnels. Un avantage important d'un tel ligament artificiel par rapport au greffon naturel réside dans la facilité de mise en oeuvre et l'obtention d'une bonne stabilité initiale. Un inconvénient sérieux résulte du caractère non réhabitable d'un tel implant. En effet en l'absence de la régénération permanente qui se produit avec un tissu vivant, les fibres du cordage s'usent et finissent par se rompre.

Le but de cette invention est de proposer un implant qui permette de concilier les avantages des deux types de solution existants, sans en présenter les inconvénients. Un tel implant devra donc être réhabitable et permettre une régénération du ligament remplacé, procurer une bonne stabilité initiale et être facile à mettre en oeuvre.

A cet effet, l'invention a pour objet un implant ligamentaire, comprenant au moins un greffon naturel disposé à l'intérieur d'une gaine réalisée en un matériau artificiel, caractérisé en ce que ladite gaine est ouverte sur au moins une partie de sa longueur et se termine à ses deux extrémités par des parties effilées semi-rigides.

Suivant d'autres caractéristiques :

– le matériau constituant la gaine est disposé à recouvrement dans la zone de l'ouverture de cette dernière;

– la gaine est réalisée sous forme d'un filet ou d'un tissu lui procurant une certaine capacité de déformation dans le sens longitudinal:

– la gaine est de préférence réalisée en un matériau résorbable.

L'invention va être décrite plus en détail ci-dessous en se référant au dessin annexé donné uniquement à titre d'exemple et sur lequel :

La Fig. 1 est une vue schématique avec arrachement d'un implant selon l'invention;

Les Figs. 2 et 3 sont des vues schématiques en coupe, de deux variantes de réalisation d'un tel implant.

On voit au dessin un implant ligamentaire 10 selon l'invention. Cet implant comprend une gaine 12 constituée par un filet ou un tissu de préférence en un matériau résorbable, se terminant à ses deux extrémités par des parties effilées semi-rigides 14.

Ces parties d'extrémité peuvent être réalisées dans le même matériau que la gaine ou bien en un matériau différent. Elles peuvent selon les cas être obtenues par soudure du matériau qui les constitue, ou par collage ou par tout autre moyen convenable.

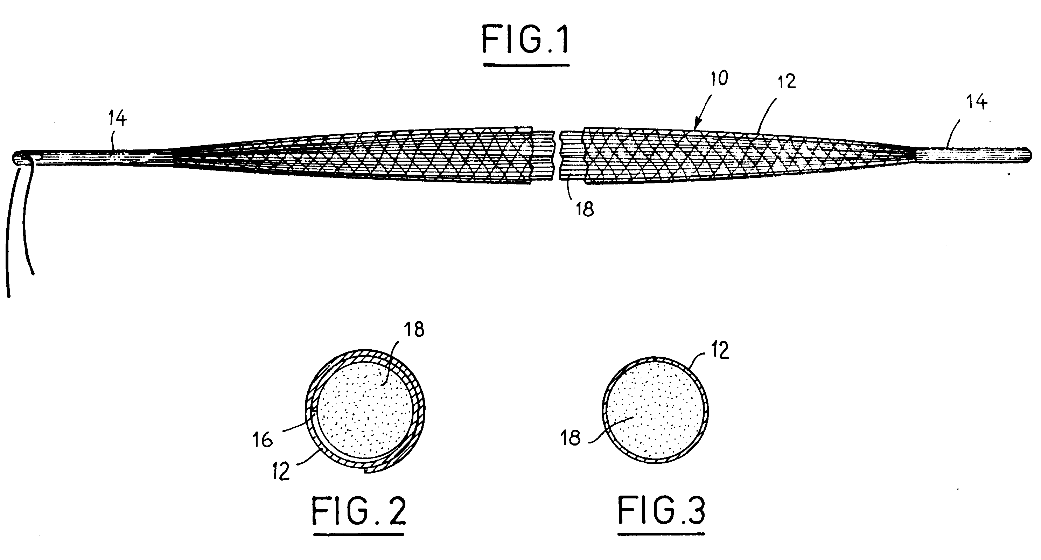

De préférence, et comme représenté au dessin (Fig. 2), la gaine est ouverte et comporte une fente 16 s'étendant sur pratiquement toute sa longueur. De plus la gaine est agencée de telle façon que le matériau qui la constitue est disposé à recouvrement au niveau de la fente précitée, ce recouvrement pouvant être de quelques dizaines de degrés ou s'étendre sur plusieurs tours, auquel cas la gaine comporte plusieurs épaisseurs.

A l'intérieur de cette gaine en matériau naturel ou synthétique, résorbable ou non, est disposé au moins un greffon naturel 18. Ce greffon peut être soit mis en place au moment de l'intervention sur le patient et peut être alors prélevé directement sur ce dernier. Il peut également être constitué par exemple, de tissu conservé, mis en place extemporanément ou déjà disposé préalablement dans la gaine, l'implant étant, dans ce dernier cas, prêt à être utilisé par le chirurgien. Il n'est pas alors nécessaire que la gaine soit fendue (Fig. 3).

En toute hypothèse l'implant formé d'une gaine et d'un greffon en tissu humain conservé doit être présenté dans un conditionnement stérile.

Les deux solutions évoquées ci-dessus peuvent d'ailleurs être combinées : une gaine fendue entoure alors un greffon deshydraté stérile et le chirurgien peut au moment de l'intervention introduire dans la gaine un greffon prélevé sur le patient. Une telle solution mixte présente comme avantage de n'obliger à prélever qu'un greffon de faible largeur.

Un tel implant résout parfaitement le problème évoqué en tête de ce mémoire. En effet :

– la gaine en matériau synthétique se terminant à ses deux extrémités par des parties effilées semi-rigides est facile à mettre en place, en particulier en ce qui concerne son introduction dans les tunnels trans-osseux;

– cette même gaine fournit une résistance initiale suffisante pendant la période pendant laquelle le greffon se fixe progressivement sur les os;

– le fait de disposer une gaine autour du greffon réalise une sorte de moulage de ce dernier et élimine ainsi tout risque d'effilochage, sans qu'il soit nécessaire d'effectuer un laçage complexe qui en tout état de cause ne peut présenter la même solidité et la même résistance qu'une gaine telle qu'utilisée selon l'invention. La solidarisation entre la gaine et le greffon peut être réalisée simplement par couture, quelques points seulement étant nécessaires.

– plusieurs greffons peuvent être disposés à l'intérieur de la gaine qui assure alors la cohésion entre ces greffons distincts; la encore les greffons peuvent être cousus par quelques points;

– lorsque la gaine est fendue, le chirurgien peut utiliser des greffons prélevés sur le malade au moment de l'intervention.

Comme on l'a déjà indiqué, le matériau constituant la gaine peut être un matériau résorbable ou non, le premier étant toutefois préféré. Ce matériau peut se présenter sous la forme d'un filet ou d'un tissu souple et présentant une certaine capacité d'allongement. Cet allongement peut résulter de la structure de maille du filet ou du tissu. De préférence la diagonale des mailles est orientée sensiblement parallèlement à la direction longitudinale de l'implant pour procurer une capacité d'allongement limitée.

## Revendications

1.  Implant ligamentaire, comprenant au moins un greffon naturel (18) disposé à l'intérieur d'une gaine (12) réalisée en un matériau artificiel, caractérisé en ce que ladite gaine est ouverte en (16) sur au moins une partie de sa longueur et se termine à ses deux extrémités par des parties effilées semi-rigides (14).

2.  Implant ligamentaire suivant la revendication 1, caractérisé en ce que le matériau constituant la gaine est disposé à recouvrement dans la zone de l'ouverture (16) de cette dernière.

3.  Implant ligamentaire suivant l'une quelconque des revendications 1 à 2, caractérisé en ce que la gaine (12) est réalisée sous forme d'un filet ou d'un tissu lui procurant une certaine capacité de déformation dans le sens longitudinal.

4.  Implant ligamentaire suivant l'une quelconque des revendications 1 à 3, caractérisé en ce que la gaine (12) est de préférence réalisée en un matériau résorbable.

5.  Implant ligamentaire suivant l'une quelconque des revendications 1 à 4, caractérisé en ce que le greffon (18) est constitué au moins en partie par du tissu conservé.

6.  Implant ligamentaire suivant la revendication 5, caractérisé en ce que le greffon en tissu conservé est disposé préalablement dans la gaine.

7.  Implant ligamentaire suivant la revendication 5, caractérisé en ce que le greffon en tissu conservé est placé extemporanément dans la gaine.

8.  Implant ligamentaire suivant l'une quelconque des revendications 1 à 7, caractérisé en ce que le greffon naturel est au moins en partie prélevé sur le patient.

9.  Implant ligamentaire suivant l'une quelconque des revendications 5 à 7, caractérisé en ce qu'il est présenté dans un conditionnement stérile.

# FIG.1

EP 0 498 690 A1

# FIG.2

# FIG.3

Office européen
des brevets

**RAPPORT DE RECHERCHE EUROPEENNE**

Numero de la demande

EP 92 40 0192

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | US-A-4 467 478 (JURGUTIS) <br> * Colonne 2, lignes 38-49,54-56,59-63; figure 3 * | 1,3,5,6 | A 61 F 2/08 |
| A | WO-A-8 901 320 (DU PONT DE NEMOURS) <br> * Page 5, lignes 15-32; page 15, lignes 24-31; abrégé; figures 1,2,4 * | 1,3 | |
| A | US-A-3 842 441 (KAISER) <br> * Colonne 9, lignes 30-48; figures 9-13 * | 1,2 | |
| A | WO-A-8 404 669 (A.H.S.C.) <br> * Page 4, ligne 11 - page 5, ligne 13; page 9, lignes 1-12; figures 2,3 * | 1,5-7,9 | |
| A | US-A-4 584 722 (LEVY) <br> * Colonne 3, lignes 30,31; revendications 6,7; figure 3 * | 3,4 | |
| A | US-A-4 917 700 (AIKINS) | | DOMAINES TECHNIQUES RECHERCHES (Int. Cl.5) |
| A | US-A-3 176 316 (BODELL) | | A 61 F |

Le présent rapport a été établi pour toutes les revendications

| Lieu de la recherche | Date d'achèvement de la recherche | Examinateur |
|---|---|---|
| LA HAYE | 17-03-1992 | KLEIN C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
                                                     
& : membre de la même famille, document correspondant

EPO FORM 1503 03.82 (P0402)